# EUROPEAN PATENT SPECIFICATION

(11) **EP 0 876 810 B1**
(45) Date of publication and mention of the grant of the patent: **12.03.2003**
(21) Application number: 98107731.6
(22) Date of filing: 28.04.1998
(51) Int. Cl.: A61F 13/15

(54) **Absorbent article**
Absorbierender Artikel
Article absorbant

(30) Priority: 06.05.1997 JP 11553397
(43) Date of publication of application: 11.11.1998
(73) Proprietor: KAO CORPORATION, Chuo-ku Tokyo (JP)
(72) Inventor: Fukuhara, Yayoi, Haga-gun, Tochigi-ken (JP); Kawasaki, Hironori, Haga-gun, Tochigi-ken (JP); Hamajima, Mitsugu, Haga-gun, Tochigi-ken (JP); Nakanishi, Minoru, Haga-gun, Tochigi-ken (JP); Yasui, Mamoru, * (JP)
(74) Representative: Grünecker, Kinkeldey, Stockmair & Schwanhäusser Anwaltssozietät

(56) References cited:
- WO-A-95/17148
- WO-A-96/23469
- US-A- 4 397 645
- US-A- 5 542 941

## Description

This invention relates to a substantially vertically elongate absorbent article, such as a sanitary napkin, an incontinent pad, a breastfeeding pad, and the like, including a liquid-permeable top layer forming a skin contacting surface, a liquid-impermeable back layer, and a liquid-retentive absorbent layer disposed between the top layer and the back layer. More particularly, the present invention is directed to an absorbent article which is capable of exhibiting an excellent leakage preventive property and providing a comfortable wearing perception.

In general, as absorbent articles, such as a sanitary napkin, an incontinent pad, a breastfeeding pad, and the like, a substantially vertically elongate absorbent article is known to include a liquid-permeable top layer forming a skin contacting surface, a liquid-impermeable back layer, and a liquid-retentive absorbent layer disposed between the top layer and the back layer. Such an absorbent article is required to absorb/retain body fluid, such as blood, urine, and the like with no leakage.

As a technique for absorbing/retaining body fluid with no leakage, heretofore, there has been known a technique for forming a leakage preventive portion projecting towards its wearer side on each side of the absorbent article. As a technique for forming a leakage preventive portion in this way, there is a technique disclosed in Japanese Patent Unexamined Publication (KOKAI) No. 1-213402, in which an elastic member is fixed to a top sheet as a top layer at an outside location of each side of the absorbent layer in a longitudinal direction of the absorbent article and the top sheet is erected in the form of a mountain in section from a back sheet as a back layer, there is also a technique disclosed in Japanese Patent Unexamined Publication (KOKAI) No. 62-250201, in which a sheet-like barrier cuffs is erected around an absorbent core and a gasket cuffs is arranged at an outside location of the barrier cuffs, and there is a further technique disclosed in Japanese Patent Unexamined Publication (KOKAI) No. 8-117273, in which an elastic element (synthetic resin film) is arranged in such a manner as to be expanded to opposite side edge portions of the skin contacting surface with opposite ends thereof fixed, so that a central portion of the elastic element is erected by expansion and contraction of the elastic element.

US-A-4,397,645 describes a disposal absorbent article according to the preamble of claim 1. It has a liquid permeable top layer, a liquid impermeable back layer and an absorbent member interposed therebetween. The top layer and the back layer extend beyond the longitudinal side margins of the absorbent member to form leakage resistant side flaps. Within the side flaps, between the top and the back layers, a sheet-like elastic member is arranged. The elastic member extends in the longitudinal direction of the article and is intended to form elasticised leg cuffs. The elastic member is attached to the top and the back layers, respectively, in a manner so as to form pillow-like gathers of the top layer which extend in a direction transversely to the longitudinal direction of the article. The elastic member is attached to the respective layer in an extended condition, and remains flat when contracted together with the respected layers.

WO 95/17148 describes a sanitary napkins having a top sheet, a back sheet and an absorbent core. The top sheet together with the absorbent core is deformed to a convex upwards configuration which may include inverted U-shapes or W-shapes or the like, which configurations extend in the longitudinal direction of the absorbent article. These configurations are formed by at least one sheet-like elastic member which extends transversely to the longitudinal direction of the article and which is fixed to both longitudinal edges of the article. The elastic member remains flat, and is not part of the shaped configuration.

US-A-5,542,941 describes an absorbent article having elasticised side flaps. The side flaps each include a loop formed by a top sheet of the article, which loop houses a string-like elastic member attached to an insert member. The string-like elastic member is not able to form a stereoscopic configuration, and remains flat when contracted.

However, the projecting leakage preventive portions disclosed in the Japanese Patent Unexamined Publications mentioned above do not satisfy the both requirements of the leakage preventing effect and the comfortable wearing feeling free from disagreeable feeling.

### SUMMARY OF THE INVENTION

It is, therefore, an object of the present invention, to provide an absorbent article which is capable of exhibiting an excellent leakage preventive property and providing a comfortable wearing perception.

The present invention has achieved the above object by providing a substantially elongate absorbent article comprising a liquid-permeable top layer forming a skin contacting surface, a liquid-impermeable back layer, and a liquid-retentive absorbent layer, interposed between the top layer and the back layer, wherein a band-like expansive sheet is arranged and fixed in the longitudinal direction of the absorbent article, and a stereoscopic configuration of a convex surface which curves towards the skin contacting surface side is formed due to contraction of the expansive sheet.

According to the present invention, an excellent leakage preventive property can be exhibited and a comfortable wearing perception can be provided.

### BRIEF DESCRIPTION OF THE DRAWINGS

Fig. 1 is a perspective view showing a sanitary napkin as one embodiment of an absorbent article of the present invention;
Fig. 2 is a sectional view taken on line I-I of Fig. 1 and viewed in a direction as indicated by arrows;
Fig. 3 is a schematic side view for explaining a concave surface configuration of the sanitary napkin 1 of Fig. 1; and
Fig. 4 is a sectional view showing another example of the embodiment of the absorbent article of the present invention.

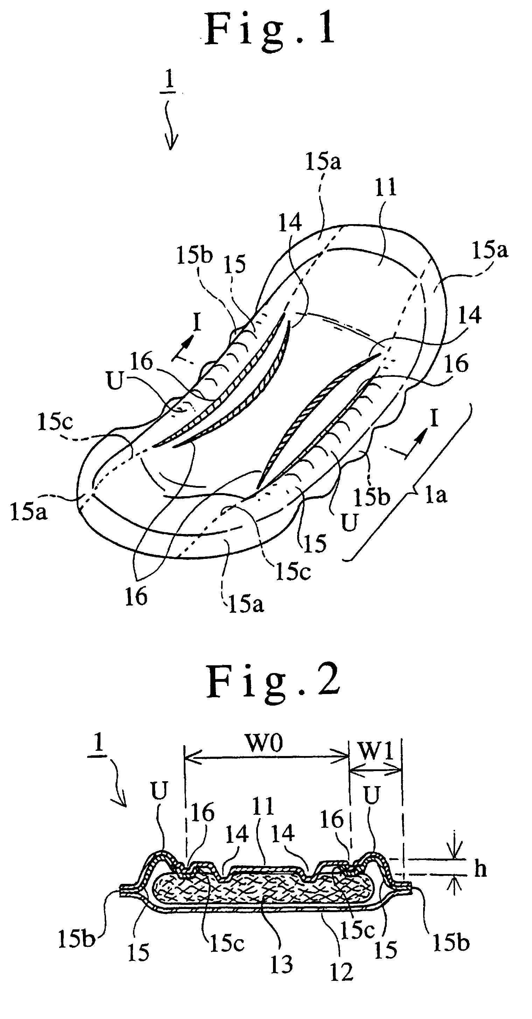

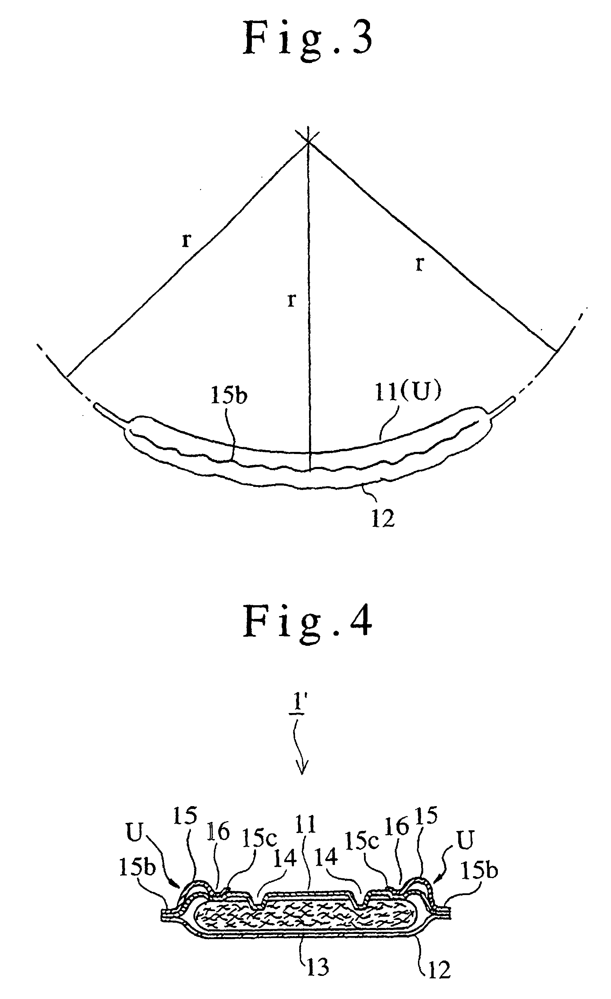

A preferred embodiment of an absorbent article of the present invention will be described specifically hereinafter with reference to the drawings. Here, Fig. 1 is a perspective view showing a sanitary napkin as one embodiment of the absorbent article of the present invention, Fig. 2 is a sectional view taken on line I-I of Fig. 1 and viewed in a direction as indicated by arrows, and Fig. 3 is a schematic side view of the sanitary napkin 1 of Fig. 1.

The sanitary napkin 1 of this embodiment comprises a top sheet 11 as a liquid-permeable top layer forming a skin contacting surface, a back sheet 12 as a liquid-impermeable back layer, and an absorbent member 13 as a liquid retentive absorbent layer interposed between the top sheet 11 and the back sheet 12. The sanitary napkin 1 has a substantially vertically elongate configuration.

Specifically, the top sheet 11 and the back sheet 12 are wider and longer than the absorbent member 13 and are bonded at an outer portion of the peripheral edge of the absorbent member 13 thereby holding the absorbent member 13 therebetween.

In the sanitary napkin 1 of this embodiment, one pair of grooves 14, 14 are formed in a skin contacting surface of a longitudinally central portion 1a along a longitudinal direction of the sanitary napkin 1 by embossing. It is designed such that the central portion 1a is readily deformed in a protruded arch configuration along the wearer at a crotch portion of the wearer, so as to be well fitted to the wearer.

This construction is the same as the prior art, and conventionally used ones can be used as the top sheet 11, the back sheet 12, and the absorbent member 13, respectively, with no limitation in particular.

The characteristic portion of this embodiment will now be described. As shown in Fig. 1 or 2, in the sanitary napkin 1 of this embodiment, band-like expansive sheets 15, 15 each are arranged and fixed in the longitudinal direction of the sanitary napkin 1, and stereoscopic configurations U, U of a convex surface which curves towards the skin contacting surface side is formed due to contraction of the expansive sheets 15, 15.

More specifically, the expansive sheets 15, 15 are arranged in opposite side portions of the sanitary napkin 1 on the skin contacting surface side located upper than the absorbent member 13 constituting the absorbent layer, and the expansive sheets 15, 15 form, in the opposite side portions of the sanitary napkin 1, and due to contraction of the expansive sheets 15, 15 stereoscopic configurations U, U of the convexly curved surface which extend each in the longitudinal direction of the sanitary napkin 1 and whose sectional shape each in the width direction is convexly arcuate towards the skin contacting surface side are formed on opposite side portions of the sanitary napkin 1.

Opposite side edges of one pair of the expansive sheets 15, 15 are fixed in a state where the expansive sheets 15, 15 are expanded in the longitudinal direction of the sanitary napkin 1.

The aforementioned expansive sheets 15, 15 are disposed over the area between one end portion of the sanitary napkin 1 and the other end portion thereof. In a state where the expansive sheets 15, 15 are expanded 30 % in the longitudinal direction of the sanitary napkin 1, they are fixed to a skin non-contacting side of the top sheet 11.

Each of the aforementioned expansive sheets 15, 15 is fixed in the longitudinal direction and in a width direction of the sanitary napkin 1, that is, opposite side edge portions 15b, 15c of the one pair of expansive sheets 15, 15 along the longitudinal direction thereof, and opposite end portions 15a, 15a of the one pair of expansive sheets 15, 15 along the width direction thereof are fixed to that surface of the top sheet 11 located on the skin non-contacting surface side thereof.

More specifically, the outer edge portions 15b, 15b arranged sidewardly and outwardly of the sanitary napkin 1 are bonded to the surface of the top sheet 11 on the skin non-contacting surface side thereof at sideward and outward areas of the absorbent member 13 to thereby form side edge portions of the sanitary napkin 1 together with the top sheet and the back sheet 12. The inner edge portions 15c, 15c arranged at those areas of the sanitary napkin 1 located inwardly of the opposite sides thereof are heat welded to the top sheet 11 and the absorbent member 13 from the skin contacting surface side of the top sheet 11 to thereby form heat-welded portions 16, 16, respectively. On the other hand, the respective end portions 15a, 15a, 15a, 15a of the one pair of expansive sheets 15, 15 along the width direction thereof are bonded to that surface of the top sheet 11 located on the skin non-contacting surface side thereof at those areas of the absorbent member 13 located outwardly of the opposite ends thereof to thereby form end portions of the sanitary napkin 1 together with the top sheet 11 and the back sheet 12.

The contracting force of the expansive sheets 15, 15 at the opposite side edges 15, 15c becomes weaker than the contracting force of the expansible sheets as a whole, and only a central portion between the opposite side edges, i.e., between the outer edge portion 15b and the inner edge portion 15c of the expansible sheet 11 is positively contracted in the longitudinal direction of the sanitary napkin 1. By this contraction, the sanitary napkin 1 is formed into a concave configuration gently curved towards the skin contacting surface side over the longitudinal direction thereof, and the above-mentioned stereoscopic configurations U, U which extend each in the longitudinal directions of the sanitary napkin 1 and whose sectional shape in the width direction is generally convexly arcuate towards the skin contacting surface side are formed on each opposite side portions of the sanitary napkin 1. And, the stereoscopic configurations U, U function as leakage preventive portions for preventing body liquid such as blood from leaking.

With respect to the manner for fixing the expansive sheet 15 in the longitudinal direction (fixing along the longitudinal direction), the outer edge portion 15b may be bonded over the entire length and the inner edge portion 15a may be bonded at the central portion 1a as in this embodiment. In the alternative, both the outer and inner edge portions 15b, 15c may be fixedly bonded over the entire length or at only the central portion 1a in the longitudinal direction.

With respect to the manner for fixing the expansive sheet 15 in the width direction (fixing along the width direction), it may be bonded at the opposite end portions as in this embodiment. In the alternative, the expansive sheet 15 may be fixed at its areas located inwardly towards the central portion 1a from the opposite end portions.

It is preferred that the expansive sheet 15 is bonded also to the absorbent member 13 as in this embodiment. If the expansive sheet 15 is bonded not only to the top sheet 11 but also to the absorbent member 13, the shape retainability of the concavely curved surface configuration and the stereoscopic configuration of the leak preventive portions U, U of the sanitary napkin 1 becomes excellent.

The expansive sheets 15, 15 are not particularly limited as far as they are in sheet form and have expansive and contractible properties. Examples of the sheet materials include an expansive film, nonwoven fabric, woven fabric and a laminated sheet thereof.

Specific examples of the sheet materials include a film whose materials per se are expansive, comprising natural rubber, polyurethane, polystyrene-polyisoprene copolymer, polystyrene-polybutadiene copolymer and polyethylene- α olefine copolymer such as ethyl-acrylate-ethylane, nonwoven fabric, woven fabric and a laminate material of the film and nonwoven fabric or the like.

Where the expansive film is used on the skin contacting surface side located upper than the top sheet, in order to give comfortable touch and improve heat sealability, it is preferred to laminate the film and thermoplastic nonwoven fabric comprising polyethylene/polyester, polyethylene/polypropylene or polypropylene for the use as the laminate material.

In the thermoplastic nonwoven fabric like this, it is preferred to use crimping fibers so that the nonwoven fabric per se may have expansive and contractible properties. In the case of laminating non-crimping nonwoven fabric which is hard to expand, only the expansive film may be expanded to the extent of a predetermined expansive rate before laminating, then bonded to the nonwoven fabric materials by means of hot melting or heat sealing whereby the expansive film gathers other layers.

The expansive sheets 15, 15 are suitably adjusted or selected in expanding property, expanding factor, fixing place, etc., so as to obtain, when the sheets 15, 15 are fixedly bonded to the sanitary napkin 1 along the longitudinal direction (15b, 15c) and the width direction (15a, 15a), a favorable curvature for forming a curved surface configuration concaved towards the skin contacting surface over the longitudinal direction and the above-mentioned stereoscopic configuration with a favorable height in the opposite side portions in the width direction.

As a curvature for forming a concavely curved surface towards the skin contacting surface side in the longitudinal direction of the sanitary napkin, the radius of curvature r (see Fig. 3) is preferably set to 50 to 300 mm, and more preferably to 100 to 200 mm. If the curvature r is smaller than 50 mm, the napkin is overly curved to deteriorate the fitness and therefore, it becomes difficult to attach the napkin to the shorts. In contrast, if the curvature is larger than 300 mm, the fitness to the body is difficult to obtain.

In the case where the convexly curved surface configuration is provided in each of the opposite side portions, the height of the stereoscopic configuration h (see Fig. 2) is preferably I to 15 mm, and more preferably 2 to 10 mm. If the height is smaller than 1 mm, a sufficiently high fitness is difficult to obtain at the side portions and therefore, the leakage preventive force is decreased. In contrast, if the height is larger than 15 mm, the disagreeable feeling will be given to the wearer.

In this way, to obtain a preferable curvature r and a preferable height h of the concavely curved surface configuration in the width direction depends on the expansive property and the expanding factor of the expansive material and the fixed points 15b, 15c of the expansive material in the width direction described below.

The expansive property of the expansive sheets 15, 15 are preferably 50 to 500 gf/ 25 mm width in stress, and more preferably 100 to 300 gf/ 25 cm width when the sheets 15, 15 are expanded 5 to 50 % in the longitudinal direction of the sanitary napkin 1.

If the stress is smaller than 50 gf /25 mm width, the expanding force is too weak to obtain a preferable curvature and to form the height h. In contrast, if the stress is larger than 500 gf / 25 mm width, the curve over the longitudinal direction becomes excessive, thus decreasing the fitness.

With respect to the fixing 15b, 15c of the expansive material, in order to obtain a desirable height h of the convexly curved surface configuration, a width W1 (see Fig. 2) between 15b and 15c is preferably 3 to 30 mm, and more preferably 5 to 20 mm. If the width W1 is smaller than 3 mm, the distance or width of the fixing area is too small to obtain a desired height h. In contrast, if the width W1 is larger than 30 mm, a width W0 of the absorptive portion becomes too small.

In this way, according to the sanitary napkin 1 of this embodiment, the stereoscopic configurations U, U of a convex surface which curves towards the skin contacting surface side are formed in the opposite side edge portions of the sanitary napkin I and function as the leakage preventive portions for preventing side leakage thereby reliably preventing the leakage of blood or the like, particularly the side leakage thereof.

The convex surface of the stereoscopic configuration extends in the longitudinal direction of the sanitary napkin 1 thereby exhibiting a comfortable fit to the wearer's body and excellent effect in leakage prevention.

Since the sanitary napkin 1 such a configuration that the skin contacting surface side is concavely curved over the longitudinal direction of the sanitary napkin 1 in the longitudinal direction of the sanitary napkin 1 by contraction of the expansive sheets 15, 15, it well fits to the wearer's body along the contour thereof also in the longitudinal direction and therefore, a forward and backward leakage can also be prevented.

In the sanitary napkin 1 of this embodiment, since the leakage preventive portions U, U are formed of the expansive sheets 15, 15, the deformation of the contacting portion makes it difficult to deform the remaining portion and therefore, no space is easily produced between the sheets and the wearer. As a consequence, an excellent leakage preventive property can be exhibited.

Since the leakage preventive portions U, U are in convex and arcuate in section, comfortable feeling of touch can be given and a load of the wearer is smoothly applied to the inside of the leakage preventive portions U, U in its increased/decreased state, and the leakage preventive portions U, U are smoothly deformed in accordance with the body pressure, the wearing perception is not decreased by the deformation. In addition, since the stress to the absorbent member is dispersed, excellent leakage prevention property can be exhibited.

In the sanitary napkin 1 of this embodiment, since there is a provision of the leakage preventive portions U, U formed by the expansive sheets 15, 15 having expandability in the longitudinal direction of the sanitary napkin 1, a comfortable perception of contact to the wearer's body can be ensured also from this point.

Since the expansive sheets 15, 15 are arranged at location towards the skin non-contacting surface side from the top sheet and the skin contacting surface is constituted by the top sheet 11, a comfortable perception of contact to the wearer's skin can be obtained irrespective of the material of the expansive sheets 15, 15.

The present invention should not be limited to the above embodiment. The specific configuration, size, etc. of each component part can appropriately be modified without departing from the spirit of the present invention.

For example, as shown in Fig. 4, it is accepted that the expansive sheets 15, 15 are arranged on the skin contacting surface side of the top sheet 11 and bonded to that surface of the top sheet 11 located on the skin contacting surface side, so that the stereoscopic configurations (leakage preventive portions U, U) are formed by both the expansive sheets 15, 15 and the top sheet 11. By this sanitary napkin 1', the same function and effect as the above-mentioned first embodiment can also be obtained. In this case, where the top sheet has a width to the fixing portions 15c, which are located inner side of the opposite side ends, the same stereoscopic configurations can be obtained, even if the top sheet does not extend till the opposite side ends 15b.

The convexly curved stereoscopic configuration may be formed in the opposite side portions of the sanitary napkin 1 by arranging and fixing the pair of expansive sheets 15, 15 to the back side of the absorbent member 13.

Furthermore, the convexly stereoscopic configuration curved towards the skin contacting surface side may be formed in the central portion of the sanitary napkin by arranging and fixing a single expansive sheet in the central portion of the sanitary napkin over the longitudinal direction.

According to the sanitary napkin thus constituted, the stereoscopic configuration fits the discharging portion of the wearer thereby effectively preventing liquid leakage. In this case, the expansive sheet may be bonded to the top sheet from the skin non-contacting surface side thereby forming convexly curved stereoscopic configuration comprising the top sheet and the expansive sheet, or the expansive sheet may be arranged and fixed to the back side of the absorbent member thereby forming the convexly curved stereoscopic configuration comprising the top sheet, absorbent member and expansive sheet.

The expansive sheets 15, 15 may be bonded to the top sheet 11 or other members by supersonic, an adhesive or the like, other than the above-mentioned heat welding.

The top layer and the absorbent layer may be integrally formed. In case the expansive sheets are arranged on the skin contacting surface, the top layer and the absorbent layer may also be integrally formed.

It is also accepted that wings are provided on the absorbent article 1, 1' on its back sheet 12 side, so that the sanitary napkin can be fixedly attached by the wings. It is also an interesting alternative that the expansive sheets 15, 15 are expanded in the width direction to thereby form expandable wings.

The absorbent article of the present invention can be applied not only to the sanitary napkin but also to an incontinent pad, a breastfeeding pad, a surgical pad, etc.

## Claims

1. A substantially elongate absorbent article (1) comprising a liquid-permeable top layer (11) forming a skin contacting surface, a liquid-impermeable back layer (12), and a liquid retentive absorbent layer (13), interposed between said top layer (11) and said back layer (12) wherein a band-like expansive sheet (15) is arranged and fixed in the longitudinal direction of said absorbent article (1), **characterized in that** due to contraction of said expansive sheet (15) said expansive sheet (15) itself is formed to a stereoscopic configuration (U) of a convex surface which extends in said longitudinal direction of said absorbent article (1) and which curves towards the skin contacting surface side.

2. The absorbent article according to claim 1, wherein said expansive sheet (15) is arranged in opposite side portions of said absorbent article (1) on the skin contacting surface side located upper than said absorbent layer (13) and said stereoscopic configuration (U) of convexly curved surface is formed on each of said opposite side portions of said absorbent article (1).

3. The absorbent article according to claim 1, wherein said expansive sheet (15) is fixed at opposite side edges (15b, 15c) thereof in a stated expanded in the longitudinal direction of said absorbent article (1), and said stereoscopic configuration (U) of convexly curved surface is formed due to contraction of said expansive sheet (15).

4. The absorbent article according to claim 1, wherein said top layer (11) comprises a top sheet, said expansive sheet (15) is fixed at least to said top sheet (11), and said convexly curved configuration extends in the longitudinal direction of said absorbent article (1).

5. The absorbent article according to claim 1, wherein said absorbent article (1) has such a configuration that said skin contacting surface side is concavely curved over the longitudinal direction of said absorbent article (1).

6. The absorbent article of any one of claims 1 to 5, wherein said expansive sheet (15) is fixed to the skin non-contacting surface of said top layer (11) between said top layer (11) and said absorbent layer (13).

7. The absorbent article of any one of claims 1 to 6, wherein said expansive sheet (15) Is bonded also to said absorbent layer (13).

8. The absorbent article of claim 7, wherein said expansive sheet (15) is bonded to said top layer (11) and said absorbent layer (13) by a heat welded portion (16) from the skin-contacting surface side of the top layer (11).

9. The absorbent article of any one of claims 1 to 8, wherein said top layer (11) and said back layer (12) are bonded to each other at an outer portion of the peripheral edge of said absorbent layer (13), with one longitudinal edge (15b) of said expansive sheet (15) bonded to said peripheral edge and an opposite edge (15c) of said expansive sheet (15) bonded to said top layer (11).

10. The absorbent article of any one of claims 1 to 9, wherein the expansive sheet (15) is fixed to the top layer (11), and the top layer (11) forms a stereoscopic configuration (U) of a convex surface together with the expansive sheet (15) due to contraction of said expansive sheet (15).

## Patentansprüche

1. Ein im Wesentlichen langgestreckter, absorbierender Artikel (1) mit einer flüssigkeitsdurchlässigen Oberschicht (11), die eine hautkontaktierende Oberfläche bildet, einer flüssigkeitstundurchlässigen, rückwärtigen Schicht (12) und einer flüssigkeitsrückhaltenden, absorbierenden Schicht (13), die zwischen der Oberschicht (11) und der rückwärtigen Schicht (12) angeordnet ist, wobei eine bandförmige, expansionsfähige Lage (15) in Längsrichtung des absorbierenden Artikels (1) angeordnet und befestigt ist, **dadurch gekennzeichnet, dass** durch die Kontraktion der expandierbaren Lage (15) die expandierbare Lage (15) selbst in eine dreidimensionale Ausbildung (U) einer konvexen Oberfläche geformt wird, die sich in Längsrichtung des absorbierenden Artikels (1) erstreckt, und die sich in Richtung auf die hautkontaktierende Oberflächenseite krümmt.

2. Absorbierender Artikel nach Anspruch 1, wobei die expansionsfähige Lage (15) in gegenüberliegenden Seitenbereichen des absorbierenden Artikels (1) auf der hautkontaktierenden Oberflächenseite angeordnet und oberhalb der absorbierenden Schicht (13) positioniert ist, und wobei die dreidimensionale Ausbildung (U) der konvexgekrümmten Oberfläche an jeder der gegenüberliegenden Seitenbereiche des absorbierenden Artikels (1) ausgebildet ist.

3. Absorbierender Artikel nach Anspruch 1, wobei die expandierbare Lage (15) an ihren gegenüberliegenden Seitenkanten (15b, 15 c) in einem Zustand befestigt ist, in dem sie in Längsrichtung des absorbierenden Artikels (1) expandiert ist, und wobei die dreidimensionale Ausbildung (U) der konvexgekrümmten Oberfläche durch die Kontraktion der expandierbaren Lage (15) gebildet ist.

4. Absorbierender Artikel nach Anspruch 1, wobei die Deckschicht (11) eine Decklage umfasst, wobei die expandierbare Lage (15) mindestens an der Decklage (11) befestigt ist, und wobei sich die konvexgekrümmte Ausbildung in Längsrichtung des absorbierenden Artikel (1) erstreckt.

5. Absorbierender Artikel nach Anspruch 1, wobei der absorbierende Artikel (1) eine derartige Ausbildung hat, dass die hautkontaktierende Oberflächenseite konkav über die Längsrichtung des absorbierenden Artikels (1) gekrümmt ist.

6. Absorbierender Artikel nach einem der Ansprüche 1-5, wobei die expandierbare Lage (15) an der die Haut nicht kontaktierenden Oberfläche der Oberschicht (11) zwischen der Oberschicht (11) und der absorbierenden Schicht (13) befestigt ist.

7. Absorbierender Artikel nach einem der Ansprüche 1-6, wobei die expandierbare Lage (15) auch mit der absorbierenden Schicht (13) verbunden ist.

8. Absorbierender Artikel nach Anspruch 7, wobei die expandierbare Lage (15) mit der Oberschicht (11) und der absorbierenden Schicht (13) durch einen Heißverschweißbereich (16) von der hautkontaktierenden Oberflächenseite der Oberschicht (11) her verbunden ist.

9. Absorbierender Artikel nach einem der Ansprüche 1-8, wobei die Oberschicht (11) und die rückwärtige Schicht (12) miteinander an einem äußeren Bereich der Umfangskante der absorbierenden Schicht (13) verbunden sind, wobei eine Längskante (15b) der expandierbaren Lage (15) mit der Umfangskante und eine gegenüberliegende Kante (15c) der expandierbaren Lage (15) mit der Oberschicht (11) verbunden ist.

10. Absorbierender Artikel nach einem der Ansprüche 1-9, wobei die expanierbare Lage(15) an der Deckschicht (11) befestigt ist, und wobei die Deckschicht (11) eine dreidimensionale Ausbildung (U) einer konvexen Oberfläche zusammen mit der expandierbaren Lage (15) durch die Kontraktion der expandierbaren Lage (15) bildet.

## Revendications

1. Article absorbant sensiblement allongé (1) comprenant une couche supérieure perméable aux liquides (11) formant une surface de contact avec la peau, une couche inférieure imperméable aux liquides (12), et une couche absorbante retenant les liquides (13), intercalée entre ladite couche supérieure (11) et ladite couche inférieure (12), dans lequel une feuille expansive en forme de bande (15) est disposée et fixée dans la direction longitudinale dudit article absorbant (1), **caractérisé en ce que**, en raison de la contraction de ladite feuille expansive (15), ladite feuille expansive (15) proprement dite prend la forme d'une configuration stéréoscopique (U) d'une surface convexe qui s'étend dans ladite direction longitudinale dudit article absorbant (1) et qui s'incurve du côté de la surface de contact avec la peau.

2. Article absorbant selon la revendication 1, dans lequel ladite feuille expansive (15) est disposée selon des portions latérales opposées dudit article absorbant (1) du côté de la surface de contact avec la peau placée au-dessus de ladite couche absorbante (13) et ladite configuration stéréoscopique (U) de la surface incurvée de manière convexe est formée sur chacune desdites portions latérales opposées dudit article absorbant (1).

3. Article absorbant selon la revendication 1, dans lequel ladite feuille expansive (15) est fixée à des bords latéraux opposés (15b, 15c) de celui-ci dans un état expansé dans la direction longitudinale dudit article absorbant (1), et ladite configuration stéréoscopique (U) de la surface incurvée de manière convexe est formée en raison de la contraction de ladite feuille expansive (15).

4. Article absorbant selon la revendication 1, dans lequel ladite couche supérieure (11) comprend une feuille de couverture, ladite feuille expansive (15) est fixée au moins à ladite feuille de couverture (11) et ladite configuration incurvée de manière convexe s'étend dans la direction longitudinale dudit article absorbant (1).

5. Article absorbant selon la revendication 1, dans lequel ledit article absorbant (1) présente une configuration telle que ledit côté de surface de contact avec la peau est incurvé de manière concave dans la direction longitudinale dudit article absorbant (1).

6. Article absorbant selon l'une quelconque des revendications 1 à 5, dans lequel ladite feuille expansive (15) est fixée à la surface non en contact avec la peau de ladite couche supérieure (11), entre ladite couche supérieure (11) et ladite couche absorbante (13).

7. Article absorbant selon l'une quelconque des revendications 1 à 6, dans lequel ladite feuille expansive (15) est collée également à ladite couche absorbante (13).

8. Article absorbant selon la revendication 7, dans lequel ladite feuille expansive (15) est collée à ladite couche supérieure (11) et à ladite couche absorbante (13) par une portion thermoscellée (16) du côté de la surface de contact avec la peau de la couche supérieure (11).

9. Article absorbant selon l'une quelconque des revendications 1 à 8, dans lequel ladite couche supérieure (11) et ladite couche inférieure (12) sont collées l'une à l'autre sur une portion extérieure du bord périphérique de ladite couche absorbante (13), un bord longitudinal (15b) de ladite feuille expansive (15) étant collé audit bord périphérique et un bord opposé (15c) de ladite feuille expansive (15) étant collé à ladite couche supérieure (11).

10. Article absorbant selon l'une quelconque des revendications 1 à 9, dans lequel la feuille expansive (15) est fixée à la couche supérieure (11), et la couche supérieure (11) forme une configuration stéréoscopique (U) d'une surface convexe avec la feuille expansive (15) en raison de la contraction de ladite feuille expansive (15).
